Europäisches Patentamt

European Patent Office

Office européen des brevets

(11) Publication number: 0 314 281 A1

# EUROPEAN PATENT APPLICATION

(21) Application number: 88308414.7

(51) Int. Cl.⁴: A61M 25/00

(22) Date of filing: 12.09.88

(30) Priority: 11.09.87 ZA 876826

(43) Date of publication of application:
03.05.89 Bulletin 89/18

(84) Designated Contracting States:
AT BE CH DE ES FR GB GR IT LI LU NL SE

(71) Applicant: Belfort, Michael Anthony
A6 Quendon Mews Quendon Road
Seat Point Cape Province(ZA)

Applicant: VEN PRODUCTS (PTY) LIMITED
910 Third Street
Marlboro Sandton Transvaal Province(ZA)

(72) Inventor: Belfort, Michael Anthony
A6 Quendon Mews Quendon Road
Seat Point Cap Province(ZA)

(74) Representative: Barlow, Roy James et al
J.A.KEMP & CO. 14, South Square Gray's Inn
London WC1R 5EU(GB)

(54) Catheter.

(57) A catheter (12) comprises a tube having a first, large diameter portion (18) and a second, small diameter portion (20). The tube portion (18) has lateral openings (26) with a diameter of at least 4mm therein and is, in use, inserted into the body cavity (32) of a patient. The tube portion (20) in use leads through the patient's abdominal wall to the outside of the abdominal cavity. A trocar (14) is provided to assist with insertion. There is a valve (16) in the tube portion (20). A "Dacron" collar (24) provides a seat for the tissue through which the catheter (12) extends when in use. The catheter (12) is particularly suitable for use in the treatment of patients suffering from malignant ascites.

FIG. I

## CATHETER

THIS INVENTION relates to a catheter. More particularly, the invention relates to a catheter for draining the abdominal cavity of a patient suffering from malignant ascites, although it is to be understood that the invention could also find application in the treatment of other forms of ascites.

A known method of draining fluids which collect in the abdomen of a patient suffering from malignant ascites is to convey the fluids to a large vein of the patient via what is called a "Le Veen shunt". This is not entirely satisfactory, as the complications of this procedure preclude its frequent, or successful application in many instances.

Conventional catheters, such as the ones used in the treatment of renal failure, consist of a relatively thin (about 2,5 mm internal diameter) tube having a number of lateral openings in the wall thereof, near the tip end of the tube which, in use, will be disposed in the abdominal cavity of the patient. To assist in inserting the catheter into the abdominal cavity, use is made of a wire-like trocar which extends the entire length of the catheter tube, through the passage or lumen thereof.

As the risk of infection increases with the diameter of the catheter tube, it is desirable to keep the diameter as small as possible. The diameter of the catheter tube in turn limits the size of the lateral openings. In most cases where there is the need for a catheter, the fluids flowing through the lateral openings is clear, so that blockage or occlusion is not a problem and the small size of the lateral openings does not matter. The fluids which collect in patients suffering from malignant ascites, however, usually contain lumps of cells, blood clots, and so on, which would rapidly occlude the lateral openings of a conventional catheter. Accordingly, catheters have heretofore not been thought of as suitable instruments for draining the abdominal cavity of patients suffering from malignant ascites.

It is an object of the present invention to provide a catheter which is less prone to occlusion and is suitable, in particular, for use in treating patients suffering from malignant ascites.

According to the invention there is provided a catheter which comprises a tube having a first portion which has lateral openings therein and which, in use, will be disposed in a body cavity of a patient, and a second portion which is in fluid flow communication with the first portion and which, in use, will be disposed on the outside of the body cavity, the minimum internal dimension, in cross section, of the first portion being substantially greater than that of the second portion.

The lateral openings preferably have a minimum internal dimension, in cross section, which is equal to or greater than that of said second portion of the tube.

Said first portion of the tube may have an internal diameter of at least 4mm, and the lateral openings may have a diameter of at least 2mm.

Said first portion of the tube may be provided externally, adjacent its junction with said second portion of the tube, with a collar of "Dacron" or the like surgically acceptable fabric material.

Said second portion of the tube may be provided with an openable closure device.

The invention extends to a catheter kit which comprises a catheter as defined above, and a trocar which extends into the lumen of said first portion of the tube via one of the lateral openings.

The pointed tip of the trocar may protrude from the end of said first portion of the tube, the kit further comprising a removable cap-like protector for covering the pointed tip.

The invention will now be described in more detail, by way of example, with reference to the accompanying drawings.

In the drawings :

Figure 1 is a side view of a catheter kit in accordance with the invention;

Figure 2 is a section through the catheter kit; and

Figure 3 shows the catheter in position in the abdominal wall of a patient.

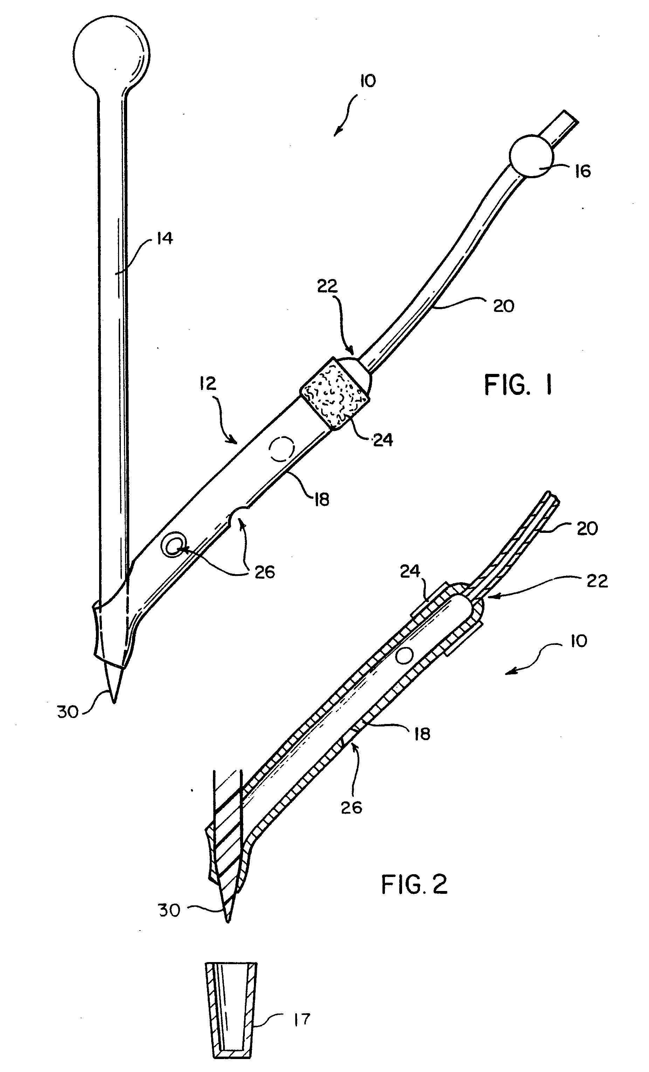

Referring first to Figures 1 and 2, reference numeral 10 generally indicates a catheter kit comprising a catheter 12, a trocar 14, a closure device 16 for closing the catheter, and a cap-like protector 17 for covering the pointed tip of the trocar 14. The entire kit may be enclosed in a sterile envelope (not shown).

The catheter 12 comprises a tube having a first, large diameter portion 18, and a second, small diameter portion 20, the two portions 18 and 20 being connected at 22 so as to be in flow communication with one another. Both portions of the tube are of a polymeric silicone substance such as that available in the trade as "Silastic". On the outside of the large diameter portion 18, near the connection 22, there is a collar 24 of a suitable fabric material such as that available in the trade as "Dacron".

The large diameter portion 18 has an inside diameter of about 6 mm. A number of lateral openings 26 extend through the wall of the portion 18. The openings 26 taper down inwardly, having a minimum diameter, where they enter into the lumen of the tube portion 18, of about 4 mm. The openings 26 are spaced apart by a distance of about 2 cm along the length of the tube portion 18 and are distributed around the circumference. The length of the tube portion 18 is not critical. In the example here described the length is about 11 cm, there being four of the openings 26.

The small diameter portion 20 has an internal diameter of about 2,5 mm.

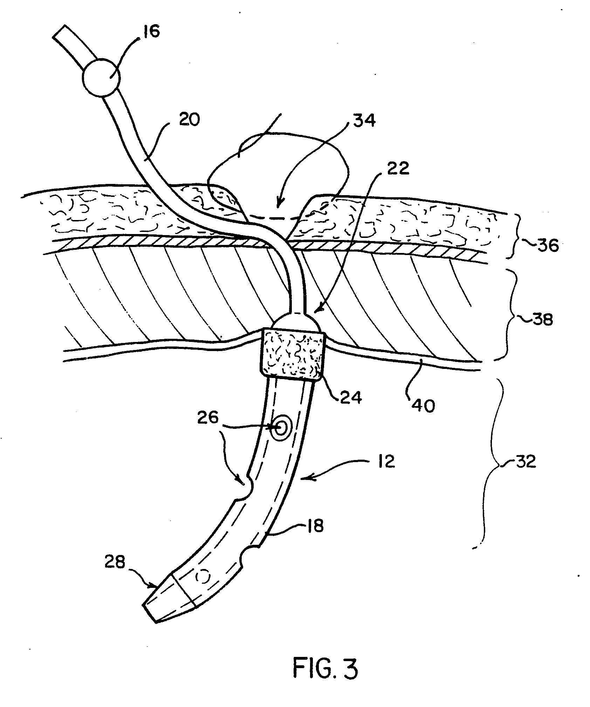

At the tip of the tube portion 18, the wall of the tube is chamfered on the outside as indicated at 28 (Figure 3), so as to facilitate insertion through the abdominal wall of a patient.

The trocar 14 enters the tube portion 18 through the lateral opening 26 which is nearest the tip, with the pointed end 30 of the trocar protruding from the mouth of the catheter. As the catheter is of an elastomeric material and the trocar somewhat distends relatively stiff material, the trocar somewhat distends the opening 26 through which it extends, and causes the tube portion 18 to bend.

Referring now to Figure 3, to insert the catheter 12 into the peritoneal or abdominal cavity 32 of a patient, an incision 34 is first made in the subcutaneous tissue 36 of the patient's abdominal wall, to expose the muscle tissue 38. The muscle tissue 38 is then split and the peritoneum 40 punctured by making use of the trocar 14, while in position in the tube portion 18 as illustrated in Figure 1 and 2. The trocar thus carries the tube portion 18 with it into the abdominal cavity 32.

The trocar 14, having now served its purpose, is withdrawn and the tube portion 18 pushed in further until it is entirely inside the abdominal cavity 32. It is then pulled out again slightly until the peritoneum 40 seats on the "Dacron" collar 24, as illustrated in Figure 3.

The protruding end of the tube portion 20 is now tunneled through the subcutaneous tissue 36 as illustrated in Figure 3, and the incision 34 closed.

The tube portion 20 will normally be closed by the closure device 16. If desired, the patient or nurse can however relieve pressure inside the abdominal cavity 32 by opening the closure device 16, so that fluids which have collected in the cavity 32 can flow via the openings 26 and the tube portion 20 into a suitable receptacle. After completion of such a drainage operation, the tube portion 20 will again be closed by means of the closure device 16.

Should occlusion of the catheter 12 occur, a sterile saline solution can be pumped via the catheter into the abdominal cavity 32, i.e. in a direction opposite to the direction of normal flow. This will usually clear the occlusion.

## Claims

1. A catheter which comprises a tube having a first portion (18) which has lateral openings (26) therein and which, in use, will be disposed in a body cavity of a patient, and a second portion (20) which is in fluid flow communication with the first portion and which, in use, will be disposed on the outside of the body cavity, characterised in that the minimum internal dimension, in cross section, of the first portion is substantially greater than that of the second portion.

2. A catheter as claimed in claim 1, characterized in that the lateral openings (26) have a minimum internal dimension, in cross section, which is equal to or greater than that of said second portion (20) of the tube.

3. A catheter as claimed in claim 1 or claim 2, characterized in that said first portion (18) of the tube has an internal diameter of at least 4mm, and wherein the lateral openings (26) have a diameter of at least 2mm.

4. A catheter as claimed in any one of the preceding claims, characterized in that said first portion (18) of the tube is provided externally, adjacent its junction with said second portion (20) of the tube, with a collar (24) of "Dacron" or the like surgically acceptable fabric material.

5. A catheter as claimed in any one of the preceding claims, characterised in that said second portion (20) of the tube is provided with an openable closure device (16).

6. A catheter kit which comprises a catheter as claimed in any one of the preceding claims, and a trocar (14) which extends into the lumen of said first portion (18) of the tube via one of the lateral openings (26).

7. A catheter kit as claimed in claim 6, characterised in that the trocar (14) has a pointed tip (30) which protrudes from the end of said first portion (18) of the tube, the kit further comprising a removable cap-like protector (17) for covering the pointed tip.

FIG. 1

FIG. 2

FIG. 3

## DOCUMENTS CONSIDERED TO BE RELEVANT

Application number

EP 88308414.7

| Category | Citation of document with indication, where appropriate, of relevant passages | Relevant to claim | CLASSIFICATION OF THE APPLICATION (Int. Cl.4) |
|---|---|---|---|
| X | US - A - 4 068 664 (R. SHARP et al.) | 1 | A 61 M 25/00 |
| Y | * Fig. 2,6,8; column 1, | 4,5 | |
| A | lines 6-16; column 3, lines 5-35; column 4, lines 38-56; column 4, line 64 - column 5, line 2 * | 2,3 | |
| | -- | | |
| Y | EP - A1 - 0 022 370 (AMERICAN HOSP.) | 4 | |
| A | * Fig. 1,2,4; page 3, line 11 - page 4, line 3; page 4, line 27 - page 5, line 14; page 5, line 36 - page 6, line 6 * | 3 | |
| | -- | | |
| Y | GB - A - 1 601 223 (R. POPOVICH) | 4,5 | |
| | * Fig. 1; page 3, lines 22- 41 * | | TECHNICAL FIELDS SEARCHED (Int. Cl.4) |
| | -- | | A 61 M 1/00 |
| X | US - A - 4 141 360 (T. LASSWELL) | 1 | A 61 M 25/00 |
| | * Fig. 1,2,3,5,9; column 3, line 31 - column 4, line 24 * | | A 61 M 27/00 |
| | -- | | |
| A | US - A - 3 459 188 (M. ROBERTS) | 6,7 | |
| | * Totality * | | |
| | ---- | | |

The present search report has been drawn up for all claims

| Place of search | Date of completion of the search | Examiner |
|---|---|---|
| VIENNA | 10-02-1989 | LUDWIG |